# EUROPEAN PATENT SPECIFICATION

(11) **EP 3 226 921 B1**
(45) Date of publication and mention of the grant of the patent: **19.08.2020**
(21) Application number: 15808312.1
(22) Date of filing: 30.11.2015
(51) Int. Cl.: A61K 31/00, A61K 33/00, A61L 15/00, A61K 31/14, A61K 31/155, A61K 31/785, A61K 33/34, A61K 33/38, A61P 31/04, A61L 26/00, A61K 33/22

(54) **ANTIMICROBIAL COMPOSITIONS COMPRISING BIOGLASS**
ANTIMIKROBIELLE ZUSAMMENSETZUNGEN UMFASSEND BIOGLAS
COMPOSITIONS ANTIMICROBIENNES COMPRENANT DU VERRE BIO

(30) Priority: 04.12.2014 US 201462087270 P
(43) Date of publication of application: 11.10.2017
(73) Proprietor: 3M Innovative Properties Company, St. Paul, MN 55133-3427 (US)
(72) Inventor: BEEN, Raha A., Saint Paul, Minnesota 55133-3427 (US); KARIM, Naimul, Saint Paul, Minnesota 55133-3427 (US); KOHLER RIEDI, Petra L., Saint Paul, Minnesota 55133-3427 (US); VAIL, Andrew W., Saint Paul, Minnesota 55133-3427 (US); VEERARAGHAVAN, Badri, Saint Paul, Minnesota 55133-3427 (US)
(74) Representative: Vossius & Partner Patentanwälte Rechtsanwälte mbB
(86) International application number: PCT/US2015/062911
(87) International publication number: WO 2016/089731

(56) References cited:
- CN-A- 101 884 807
- CN-A- 103 705 968
- CN-A- 103 736 135
- HAO DING ET AL: "A Novel Injectable Borate Bioactive Glass Cement as an Antibiotic Delivery Vehicle for Treating Osteomyelitis", PLOS ONE, vol. 9, no. 1, 10 January 2014 (2014-01-10), page e85472, XP055247169, DOI: 10.1371/journal.pone.0085472
- SHI-HUA LUO ET AL: "In vitro evaluation of cytotoxicity of silver-containing borate bioactive glass", JOURNAL OF BIOMEDICAL MATERIALS RESEARCH. PART B: APPLIED BIOMATERIALS, vol. 95B, no. 2, 28 September 2010 (2010-09-28), pages 441-448, XP055247186, US ISSN: 1552-4973, DOI: 10.1002/jbm.b.31735

## Description

### CROSS REFERENCE TO RELATED APPLICATIONS

This application claims priority to U.S. Provisional Patent Application No. 62/087,270, filed December 4, 2014.

### BACKGROUND

When an injury occurs, cell damage initially comes from the precipitating event, such as a cut, resulting in ruptured cells and severed or crushed capillaries and other blood vessels. However, later damage can occur due to bacterial growth or to an inflammatory response.

An excessive inflammatory response can cause extensive swelling, which can lead to additional injury as a result of anoxia. The quantity of bacterial burden in a wound bed is also an important factor in the healing of wounds, especially dermal ulcers. Some bacterial colonization is inevitable, and may even be beneficial in stimulating the body's natural immune response. However, excessive bacterial colonization is clearly detrimental and can lead to high levels of bacterial waste products, chronic inflammation, heavy exudate, increased tissue necrosis and eventually, full infection. Wounds typically will not heal when the bacterial burden is above about 10⁵ microorganisms per gram of tissue.

Topical anti-microbial agents, including organism-specific antibiotics such as bacitracin and silver sulfadiazine, are typically used in wound care. However, the effects of these agents are regarded as relatively weak. More importantly, the recent rise of strains of microorganisms resistant to these agents has led to many intractable cases of infection. Other typically-used antimicrobial agents, such as iodine and alcohol, damage native tissue and repair cells, and retard the healing process of dermal wounds.

Treatments have been proposed for treating wounds to accelerate wound healing. Such treatments involving the use of bioactive glass composition, growth factors such as platelet derived growth factor (PDGF), or the use of cultured cells derived from the wounded patient's own skin. Further, bioactive glass compositions with antimicrobial cations such as silver, have been used for wound dressing and surface treatment (see e.g. CN 103705968 A and CN 101884807 A).

There remains a need for an effective treatment to control microorganisms at a wound site and thereby promote wound healing.

### SUMMARY

The present disclosure generally relates to compositions, articles, and methods for reducing or eliminating a population of microorganisms (e.g., bacteria) from a surface (of an inanimate object), as well as compositions for use in the treatment of a wound site (e.g. biological tissue such as skin or exposed wound tissue).

It is now known that compositions comprising a suspending medium, in which bioglass and certain antimicrobial compounds disclosed herein are disposed, facilitate the reduction of viable microorganisms in a biofilm. In addition, it is now known that compositions comprising a suspending medium, in which bioglass and certain antimicrobial compounds disclosed herein are disposed, inhibit formation of a biofilm and/or inhibit proliferation of viable microorganisms in a biofilm. The present disclosure provides composition and methods for treating existing biofilms and/or inhibiting formation of biofilms and/or inhibiting proliferation of microorganisms in existing biofilms.

In one aspect, the present disclosure provides a composition which comprises a suspending medium, a bioactive glass comprising B₂O₃, and an effective amount of an antimicrobial biguanide compound. In any embodiment, the antimicrobial biguanide can be selected from the group consisting of polyhexamethylene biguanide, chlorhexidine, alexidine, polyaminopropyl biguanide, a salt of any one of the foregoing antimicrobial biguanides, and a combination of any two or more of the foregoing antimicrobial biguanides.

In another aspect, the present disclosure provides a composition which comprises a suspending medium, a bioactive glass comprising B₂O₃, and an effective amount of an antimicrobial quaternary ammonium compound. In any embodiment, the antimicrobial quaternary ammonium compound can be selected from the group consisting of cetylpyridinium chloride, benzalkonium chloride, benzethonium chloride, methylbenzethonium chloride, cetalkonium chloride, cetylpyridinium chloride, cetrimonium, cetrimide, dofanium chloride, tetraethylammonium bromide, didecyldimethylammonium chloride, domiphen bromide, a salt of any one of the foregoing antimicrobial quaternary ammonium compounds, and a combination of any two or more of the foregoing antimicrobial quaternary ammonium compounds.

For reference disclosed herein is a composition comprising a suspending medium, a bioactive glass comprising B₂O₃, and an effective amount of an antimicrobial compound that comprises silver or copper. The bioactive glass has a surface. The antimicrobial compound is present in the composition as a coating on the surface of the bioactive glass. The antimicrobial compound can be selected from the group consisting of silver oxide, metallic silver, copper (II) oxide, metallic copper, and combinations thereof.

In yet another aspect, the present disclosure provides a composition which comprises a suspending medium, a bioactive glass comprising B₂O₃, and effective amount of an antimicrobial agent comprising an antimicrobial lipid component. The antimicrobial lipid component comprises a (C7-C12)saturated fatty acid ester of a polyhydric alcohol, a (C8-C22)unsaturated fatty acid ester of a polyhydric alcohol, a (C7-C12)saturated fatty ether of a polyhydric alcohol, a (C8-C22)unsaturated fatty ether of a polyhydric alcohol, an alkoxylated derivative thereof, or combinations thereof, wherein the alkoxylated derivative has less than 5 moles of alkoxide per mole of polyhydric alcohol; with the proviso that for polyhydric alcohols other than sucrose, the esters comprise monoesters and the ethers comprise monoethers, and for sucrose the esters comprise monoesters, diesters, or combinations thereof, and the ethers comprise monoethers, diethers, or combinations thereof. In any embodiment, the antimicrobial lipid component can include a compound selected from the group consisting of glycerol monolaurate, glycerol monocaprate, glycerol monocaprylate, propylene glycol monolaurate, propylene glycol monocaprate, propylene glycol monocaprylate, or combinations thereof.

In any of the above embodiments of the composition, the bioactive glass can comprise less than 35 weight percent SiO₂. In any of the above embodiments, the borate bioactive glass can be present in the composition in a portion that constitutes about 5 percent by weight to about 95 percent by weight.

In yet another aspect, the present disclosure provides a wound dressing comprising the composition of any of the above embodiments.

In yet another aspect, the present disclosure provides a method of treating a biofilm on a surface of an inanimate object.
The method comprises contacting a biofilm with any of the above embodiments of the compositions.

In yet another aspect, the present disclosure provides a method of treating a surface of an inanimate object
to inhibit formation of a microbial biofilm thereon. The method comprises contacting the surface with any of the above embodiments of the compositions.

In yet another aspect, the present disclosure provides the compositions for use in treating a wound
site. The method can comprise contacting the wound site with any of the above embodiments of the compositions. In any embodiment, contacting the wound site with the composition can comprise contacting the wound site with the composition for a period of time up to seven days.

As used herein, the term "biofilm" refers to any three-dimensional, matrix- encased microbial community. Accordingly, as used herein, the term biofilm includes surface-associated biofilms as well as biofilms in suspension, such as floes and granules. Biofilms may comprise a single microbial species or may be mixed species complexes, and may include bacteria as well as fungi, algae, protozoa, or other microorganisms.

As used herein, the term "soft tissue" refers to a tissue that connects, supports, or surrounds organs and structures of the body, and which is not bone. Examples of soft tissues include, but are not limited to, tendon tissue, ligament tissue, meniscus tissue, muscle tissue, skin tissue, bladder tissue, and dermal tissue.

As used herein, the term "pharmaceutically acceptable" refers to compounds and compositions which may be administered to mammals without undue toxicity.

As used herein, the term "suspending medium" refers to a composition of one or more compounds in which the bioglass particles and/or fibers and the antimicrobial compounds are suspended. Preferably, the bioglass particles and/or fibers and the antimicrobial compounds are uniformly dispersed in the suspending medium.

As used herein, the term "surface" is defined as any surface which may be covered, at least in part, by a biofilm. This may include surfaces of medical devices prone to biofilm formation. Examples of surfaces may include, but are not limited to, metal, plastic, rubber, glass, or any material suitable for a medical device. In any embodiment, the surface may include an internal or external surface of a medical instrument, such as, but not limited to, an endoscope, a catheter, etc. Further, in any embodiment, the surface may include a coating, such as, for example, polytetrafluoroethylene.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

The terms "comprises" and variations thereof do not have a limiting meaning where these terms appear in the description and claims.

As used herein, "a," "an," "the," "at least one," and "one or more" are used interchangeably. Thus, for example, "an" antimicrobial component can be interpreted to mean "one or more" antimicrobial components.

The term "and/or" means one or all of the listed elements or a combination of any two or more of the listed elements.

Also herein, the recitations of numerical ranges by endpoints include all numbers subsumed within that range (e.g., 1 to 5 includes 1, 1.5, 2, 2.75, 3, 3.80, 4, 5, etc.).

The above summary of the present invention is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

Additional details of these and other embodiments are set forth in the accompanying drawings and the description below. Other features, objects and advantages will become apparent from the description and drawings, and from the claims.

### DETAILED DESCRIPTION

Before any embodiments of the present disclosure are explained in detail, it is to be understood that the invention is not limited in its application to the details of construction and the arrangement of components set forth in the following description or illustrated in the following drawings. The invention is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Unless specified or limited otherwise, the terms "connected" and "coupled" and variations thereof are used broadly and encompass both direct and indirect connections and couplings. Further, "connected" and "coupled" are not restricted to physical or mechanical connections or couplings. It is to be understood that other embodiments may be utilized and structural or logical changes may be made without departing from the scope of the present disclosure. Furthermore, terms such as "front," "rear," "top," "bottom," and the like are only used to describe elements as they relate to one another, but are in no way meant to recite specific orientations of the apparatus, to indicate or imply necessary or required orientations of the apparatus, or to specify how the invention described herein will be used, mounted, displayed, or positioned in use.

The present disclosure relates generally to antimicrobial compositions and methods for treating a biofilm. In addition, the present disclosure generally relates to antimicrobial compositions and methods for inhibiting or reducing the formation of a biofilm. Advantageously, the compositions of the present disclosure easily can be applied to a surface (e.g., the surface of a wound, an environmental surface), where the antimicrobial properties of the composition can facilitate the reduction of viable microorganisms and/or inhibit colonization of the surface by microorganisms. Moreover, the antimicrobial properties of the compositions of the present disclosure are exerted against microorganisms present in a biofilm.

In particular, the present disclosure relates to compositions comprising bioglass scaffolds (e.g., particles and/or fibers) and certain antimicrobial compounds disposed in a pharmaceutically-acceptable suspending medium. Advantageously, the antimicrobial compounds act in concert with the bioglass to reduce the number of viable microorganisms in a biofilm and/or inhibit the formation of a biofilm and/or inhibit the proliferation of microorganisms in a biofilm.

Introduction of a microorganism into an environment (e.g., a tissue, a surface) can result in colonization (e.g., with attendant increases in microbial numbers) of the environment. Colonization of the environment can precede or coincide with the formation of a biofilm on and/or in the environment. The establishment of a colony and/or a biofilm in the environment can alter the environment in ways that facilitate colonization and/or infection by a pathogenic microorganism. A biofilm may be formed by a pathogenic microorganism (e.g., certain species of *Pseudomonas*) or a pathogenic microorganism may be introduced into and propagate in the environment existing in or adjacent the biofilm. Thus, compositions that are capable of reducing or eliminating microorganisms associated with a biofilm can reduce or eliminate a risk of infection by a pathogenic microorganism.

Microbes (i.e., microorganisms) of particular interest include prokaryotic and eukaryotic organisms, particularly Gram positive bacteria, Gram negative bacteria, fungi, protozoa, mycoplasma, yeast, viruses, and even lipid-enveloped viruses. Particularly relevant organisms include members of the family *Enterobacteriaceae,* or the family *Micrococcaceae* or the genera *Staphylococcus* spp., *Streptococcus* spp., *Pseudomonas* spp., *Enterococcus* spp., *Salmonella* spp., *Legionella* spp., *Shigella* spp. *Yersinia* spp., *Enterobacter* spp., *Escherichia* spp., *Bacillus* spp., *Listeria* spp., *Vibrio* spp., *Corynebacteria* spp. as well as herpes virus, *Aspergillus* spp., *Fusarium* spp., and *Candida* spp. Particularly virulent organisms include *Staphylococcus aureus* (including resistant strains such as Methicillin Resistant *Staphylococcus aureus* (MRSA)), *S. epidermidis, Streptococcus pneumoniae, S. agalactiae, S. pyogenes, Enterococcus faecalis,* Vancomycin Resistant *Enterococcus* (VRE), Vancomycin Resistant *Staphylococcus aureus* (VRSA), Vancomycin Intermediate-resistant *Staphylococcus aureus* (VISA), *Bacillus anthracis, Pseudomonas aeruginosa, Escherichia coli, Aspergillus niger, A. fumigatus, A. clavatus, Fusarium solani, F. oxysporum, F. chlamydosporum, Listeria monocytogenes, Listeria ivanovii, Vibrio cholera, V. parahemolyticus, Salmonella cholerasuis, S. typhi, S. typhimurium, Candida albicans, C. glabrata, C. krusei, Enterobacter sakazakii, E. coli* 0157 and multiple drug resistant (MDR) Gram negative rods.

Gram positive and Gram negative bacteria are of particular interest. Of even more interest are certain Gram positive bacteria, such as *Staphylococcus aureus.* Also, of particular interest are antibiotic resistant microbes including MRSA, VRSA, VISA, VRE, and MDR.

A variety of different bacteria are capable of forming biofilms including, for example, *Bacillus* species, *Listeria monocytogenes, Staphylococcus* species, *Lactobacillus plantarum, Lactococcus lactis, Escherichia coli, Pseudomonas aeruginosa, Pseudomonas putida, Pseudomonas fluorescens, Rhizobium leguminosarum*, and *Sinorhizobium meliloti.*

Compositions and methods for treating wounds to significantly reduce the occurrence and/or extent of microbial colonization of a wound are disclosed. By decreasing or eliminating microbial colonization at a wound site, the compositions and methods allow wounds to heal in significantly less time than would otherwise occur.

The compositions include bioactive glass particles and/or fibers in combination with a specific antimicrobial compound. The bioactive glass is a borate bioglass (i.e., the glass comprises boron trioxide (B₂O₃). Formulations including the composition and a suitable suspending medium, preferably for topical application, are disclosed.

Not being bound to any particular theory or mechanism, it is believed that the surface area and reactivity of bioactive glass particles and/or fibers adsorb hydronium ions from an aqueous environment (e.g., wound fluid) release other ions (e.g., sodium, calcium) that increase the localized pH of the environment proximate the bioglass. This may facilitate pH-dependent binding of hemoglobin, thereby increasing the amount of oxygen in a wound or burn site, for example.

The reactions that involve bioglass and an aqueous liquid also cause a higher negative surface charge on the glass surface and the development of a high specific surface area (e.g., from 0.5 m²/g initially to over 50 m²/g by 12 hours) which may attract collagen, fibrin, fibronectin, and cells, which may facilitate wound healing. Moreover, the bioactive glass facilitates precipitation of calcium and phosphorous naturally present in the wound exudate and blood, which can cause the rapid formation of a calcium phosphate layer that may incorporate collagen, fibrin and fibronectin to stabilize the wound quickly and effectively. In addition, the bioactive glass is believed to moderate the typical inflammatory response present in chronic wounds.

The terms "wound" and "burn," collectively referred to herein as "injury" have their usual meanings. "Normal" is used in the sense it is usually used in the medical arts. The terms "antimicrobial compounds" and "antibiotics" as used herein mean pharmacologically acceptable synthetic or natural agents which destroy or inhibit microorganisms and include antibacterial, antifungal and antiviral agents. "Medical practitioner" means one of ordinary skill in the art of wound and burn treatment. Typically this person is a physician, nurse, dentist, or paramedic.

Compositions including particles and/or fibers of bioactive glass in combination with an antimicrobial compound can be used for a variety of purposes.

As used herein the terms "bioactive glass" or "biologically active glass" mean an inorganic glass material having an oxide of boron as a component and which is capable of bonding with growing tissue when reacted with physiological fluids.

Bioactive glasses are well known to those skilled in the art, and are disclosed, for example, U.S. Patent No. 6,75 6,060. In any embodiment of a composition of the present disclosure, the composition comprises an effective, antibacterial amount of bioactive glass. An "effective, antibacterial amount of bioactive glass", as used herein, refers to an amount of bioactive glass, with an appropriate particle size, which is effective at reducing the bacterial infection. Those having ordinary skill in the art can readily estimate the bacterial load in a wound, and use this estimate in view of the present disclosure to determine an appropriate quantity of the composition of the present disclosure to administer to a particular treatment site..

The glass includes boron trioxide. In any embodiment, the bioactive glass comprises between 1% and 80%, inclusive, by weight of boron trioxide (B₂O₃). In any embodiment, the bioactive glass comprises between 10% and 70%, inclusive, by weight of boron trioxide (B₂O₃). In any embodiment, the bioactive glass comprises between 15% and 60%, inclusive, by weight of boron trioxide (B₂O₃).

In any embodiment, the bioactive glass also comprises one or more oxides selected from the group consisting of silicon dioxide oxide (SiO₂), sodium oxide (Na₂O), calcium oxide (CaO), and phosphorus oxide (P₂O₅). The oxides can be present as solid solutions or mixed oxides, or as mixtures of oxides.

CaF₂, Al₂O₃, MgO and K₂O may be included in the bioglass in addition to boron, sodium, phosphorus and calcium oxides. In any embodiment, the bioglass may comprise less than about 35% by weight silicon dioxide. In any embodiment, the bioglass may comprise less than about 30% by weight silicon dioxide. In any embodiment, the bioglass may comprise less than about 25% by weight silicon dioxide. In any embodiment, the bioglass may comprise less than about 20% by weight silicon dioxide. In any embodiment, the bioglass may comprise less than about 15% by weight silicon dioxide. In any embodiment, the bioglass may comprise less than about 10% by weight silicon dioxide. In any embodiment, the bioglass may comprise less than about 5% by weight silicon dioxide. In any embodiment, the bioglass can be substantially free of silicon dioxide.

In any embodiment, antimicrobial salts such as AgNO₃, CuO, and ZnO, or other antimicrobial salts of the silver, copper and Zinc ions, such as nitrates, acetates, etc., can be present in a composition according to the present disclosure. The preferred range for these salts, when present in the composition, is between > 0% and up to 5% by weight.

In any embodiment, the bioglass used in a composition of the present disclosure has a composition that is generally known in the art as a 13-93B3 glass, which is described in an article published in Advances in Bioceramics and Porous Ceramics V ("In Vitro Evaluation of Silicate and Borate Bioactive Glass Scaffolds Prepared by Robocasting of Organic-based Suspensions"; A.M. Deliormanh and M.N. Rahaman; Volume 33, Pages 11-20; 2013). Thus, in any embodiment, the bioglass used to make a composition of the present disclosure comprises about 56.6% by weight boron trioxide (B₂O₃), about 18.5% by weight calcium oxide (CaO), about 5.5% by weight sodium oxide (Na₂O), about 11.1% by weight potassium Oxide (K₂O), about 4.6% by weight magnesium oxide (MgO), and about 3.7% by weight phosphorous pentoxide (P₂O₅).

In any embodiment, the bioactive glass used in a composition of the present disclosure comprises glass particles. In any embodiment, the glass particles may be substantially non-interlinked glass particles. That is, the glass is in the form of small, discrete particles, rather than a fused matrix of particles or a mesh or fabric (woven or non-woven) of glass fibers. Note that under some conditions the discrete particles of the present disclosure may tend to cling together because of electrostatic or other forces but are still considered to be non-interlinked. In any embodiment, the mean particle diameter is 0.5 microns to about 100 microns, as measured by SEM or laser light scattering techniques.

Highly porous bioactive glass has antimicrobial properties similar to small particles of bioactive glass, due to its relatively fast degradation rate and high surface area, in comparison to non-porous bioactive glass compositions. When highly porous bioactive glass is used in place or in addition to small particles of bioactive glass, the pore size is between about 0 and 500 µm, preferably between about 10 and 150 µm, and more preferably, between about 50 and 100 µm. The degree of porosity of the glass is between about 0 and 85%, preferably between about 30 and 80%, and more preferably, between about 40 and 60%. Porous bioactive glass can be prepared, for example, by incorporating a leachable substance into the bioactive glass composition, and leaching the substance out of the glass. Suitable leachable substances are well known to those of skill in the art, and include, for example, sodium chloride and other water-soluble salts. The particle size of the leachable substance is roughly the size of the resulting pore. The relative amount and size of the leachable substance gives rise to the degree of porosity. Also, as described herein, porosity can be achieved using sintering and/or by controlling the treatment cycle of glass gels to control the pores and interpores of the material.

In any embodiment, the bioactive glass may be provided in the composition in the form of glass fibers. The glass fibers comprise borate bioactive glass, as described herein. In any embodiment, the composition comprises a combination of bioglass particles and bioglass fibers.

The glass composition can be prepared in several ways including, for example, those described in U.S. Patent No. 6,756,060; to provide melt-derived glass, sol-gel derived glass, and sintered glass particles. The sintered particles may be in sol-gel derived, or pre-reacted melt derived form. In each preparation, it is preferred to use reagent grade glass, especially since the glass is used to prepare materials which ultimately may be administered to a patient.

The bioactive glass may be administered to the wound in a topical, pharmaceutical formulation, such as in the form of a suspension, lotion, cream, ointment, or gel. Those skilled in the art will appreciate that there are other appropriate topical suspending mediums such as those listed in U.S.P.D.

It is acceptable to place particulate bioactive glass directly into a wounded area or on a burn with no suspending medium or excipient. However, preferably bioactive glass alone or in combination with one or more other therapeutic agents is combined in any suspending medium for topical use, such as a suspension, ointment, cream, or gel to facilitate application to the wound. For example, the composition of the present invention can be blended with white petrolatum to form an ointment, with mineral oil to form a suspension, with a commercially available, cream cosmetic base to form a non-greasy cream, or with a commercially available water-soluble, lubricating gel, e.g., K Y Gel™, to form a high moisture gel.

In any embodiment, a composition of the present disclosure may comprise a suspending medium that includes two or more polyols. In any embodiment, the two or more polyols may comprise one or more poly(ethylene oxide) polyols. In any embodiment, the suspending medium may comprise, for example, glycerol or a first poly(ethylene oxide) polyol having a melting point that is less than or equal to ambient temperature (25° C). The first polyol can have a weight average molecular weight of about 400 daltons, for example. In any embodiment, the suspending medium may comprise a second poly(ethylene oxide) polyol having a melting point that is greater than ambient temperature (25° C). The second polyol can have a weight average molecular weight of about 4000 daltons.

In any embodiment, a composition according to the present disclosure can take the form of a paste or a gel (e.g., have a predetermined viscosity) at ambient temperature. The viscosity can be predetermined by selecting the suspending medium components (e.g., two or more polyols) and their respective ratios in the composition. In any embodiment, a composition of the present disclosure can have a viscosity of about 0.1 to about 100,000 Pa·s (pascal seconds) at 37 degrees C measured at a shear rate of 3.8/s in parallel plate mode.

The bioactive glass and other therapeutic agents can be combined with other wound and burn treatments or dressings such as, but not limited to, collagen, fibrin, fibronectin, various growth factors, such as PDGF, TGF-β, vitamin E, gauze, cotton, cellulose, synthetic wound or burn dressings and other wound or burn dressings/treatments known to those of ordinary skill in the art. Dressings of fiberglass, including fiberglass made from fibers of bioactive glass, can also be used. In addition, the bioactive glass may be combined with any biocompatible material, such as biodegradable polymer like polylactic/glycolic acid to form a composite material for accelerating wound healing.

While the ratio of bioactive glass to suspending medium is not critical, in any embodiment, the blend of bioactive glass, the antimicrobial compound, and suspending medium contains about 5% by weight to about 95% by weight bioactive glass. In any embodiment, the blend of bioactive glass, the antimicrobial compound, and suspending medium contains about 10% by weight to about 80% by weight bioactive glass. In any embodiment, the blend of bioactive glass, the antimicrobial compound, and suspending medium contains about 20% by weight to about 60% by weight bioactive glass. In any embodiment, the median particle diameter for the bioactive glass is about 0.5 microns to about 100 microns, as measured by laser diffraction. In any embodiment, median particle sizes specifically less than about 25 microns, as well as less than about 10 microns can also be used, as well as less than about 2 microns, where the particle sizes are measured by SEM or laser light scattering techniques. Particles having generally smaller sizes (e.g., median particle diameters less than about 20 microns) generally provide advantageous antimicrobial efficacy but do not illicit any undesirable immune response.

If the bioactive glass is to be mixed with a topical suspending medium such as an ointment, then it is preferable that the glass not be significantly pre-reacted (e.g., contacted with aqueous solutions or gels) prior to application. This can be achieved, for example, by applying the composition immediately after mixing. Alternatively, the topical suspending medium may be of such a nature as to not pre-react the glass such as, for example, glycerin and/or polyethylene glycol.

Optionally, in any embodiment, the bioactive particulate glass and topical suspending medium can be held as separate components in a two part system wherein the bioactive glass and topical suspending medium are mixed and simultaneously applied. For example, a two part mixing syringe with two separate storage chambers and a mixing chamber can be used. After mixing the separate components to form the composition, the composition can be applied to a wound or burn, followed by application of a dressing or bandage to cover the composition and wound site. Alternatively, or additionally, after mixing the separate components to form the composition, the composition can be applied to a dressing or bandage, which can be applied to the treatment (e.g., wound or burn) site. Other two part delivery systems are known to those of ordinary skill in the art.

In one aspect, the present disclosure provides a composition. The composition can be applied to a surface (e.g., the surface of a wound, an environmental surface), where the antimicrobial properties of the composition can facilitate the reduction of viable microorganisms and/or inhibit colonization of the surface by microorganisms. The composition comprises a suspending medium as described herein, a bioactive glass comprising B₂O₃ as described herein, and an effective amount of an antimicrobial biguanide compound. The amount of antimicrobial biguanide compound is effective to cause the composition to have a greater antimicrobial effect, when the composition is contacted with a biofilm comprising microorganisms (e.g., *Pseudomonas aeruginosa*), than an otherwise identical composition that does not include the antimicrobial biguanide. In any embodiment, the antimicrobial biguanide is present in the composition in a portion that constitutes about 0.01 percent by weight to about 5 percent by weight of the composition. In any embodiment, the antimicrobial biguanide is present in the composition in a portion that constitutes about 0.05 percent by weight to about 3 percent by weight of the composition. In any embodiment, the antimicrobial biguanide is present in the composition in a portion that constitutes about 0. 1 percent by weight to about 1 percent by weight of the composition.

A number of suitable antimicrobial biguanides can be used in a composition according to the present disclosure. Non-limiting examples of suitable antimicrobial biguanides include polyhexamethylene biguanide, chlorhexidine, alexidine, polyaminopropyl biguanide, a salt of any one of the foregoing antimicrobial biguanides, and a combination of any two or more of the foregoing antimicrobial biguanides.

In another aspect, the present disclosure provides a composition. The composition can be applied to a surface (e.g., the surface of a wound, an environmental surface), where the antimicrobial properties of the composition can facilitate the reduction of viable microorganisms and/or inhibit colonization of the surface by microorganisms. The composition comprises a suspending medium as described herein, a bioactive glass comprising B₂O₃ as described herein, and an effective amount of a quaternary ammonium compound. The amount of quaternary ammonium compound is effective to cause the composition to have a greater antimicrobial effect, when the composition is contacted with a biofilm comprising microorganisms (e.g., *Pseudomonas aeruginosa*), than an otherwise identical composition that does not include the quaternary ammonium compound. In any embodiment, the antimicrobial quaternary ammonium compound is present in the composition in a portion that constitutes about 0.01 percent by weight to about 5 percent by weight of the composition. In any embodiment, the antimicrobial quaternary ammonium compound is present in the composition in a portion that constitutes about 0.05 percent by weight to about 3 percent by weight of the composition. In any embodiment, the antimicrobial quaternary ammonium compound is present in the composition in a portion that constitutes about 0.1 percent by weight to about 1 percent by weight of the composition.

A number of suitable quaternary ammonium compounds can be used in a composition according to the present disclosure. Non-limiting examples of suitable quaternary ammonium compounds include cetylpyridinium chloride, benzalkonium chloride, benzethonium chloride, methylbenzethonium chloride, cetalkonium chloride, cetylpyridinium chloride, cetrimonium, cetrimide, dofanium chloride, tetraethylammonium bromide, didecyldimethylammonium chloride, domiphen bromide, and a combination of any two or more of the foregoing antimicrobial quaternary ammonium compounds.

Disclosed herein for reference is a composition. This composition can be applied to a surface (e.g., the surface of a wound, an environmental surface), where the antimicrobial properties of the composition can facilitate the reduction of viable microorganisms and/or inhibit colonization of the surface by microorganisms. The composition comprises a suspending medium as described herein, a bioactive glass comprising B₂O₃ as described herein, and an effective amount of an antimicrobial compound that comprises silver or copper, wherein the antimicrobial compound is present in the composition as a coating on the surface of the bioactive glass. Suitable antimicrobial compounds include, for example, silver oxide, metallic silver, copper (II) oxide, metallic copper, and combinations thereof.

The surface coating may be deposited using a process selected from the group consisting of a solution deposition process and vapor deposition process. The vapor deposition process can be a physical vapor deposition process as described in U.S. Patent No. 7,727,931. Advantageously, the vapor-deposited surface-coated metal or metal oxide, in contrast to a solution-coated water-soluble metal salt, does not readily dissolve into wound fluid and, thus, can provide more antimicrobial activity that is more durable (i.e., longer-lasting) than an antimicrobial metal salt. In addition, vapor deposition of antimicrobial coatings may result in particles having a high degree of surface morphology (e.g., 3-D structure). The resulting higher surface area can result in higher rates of release of the antimicrobial compound in a treatment site, longer-lasting release of the antimicrobial compound in the treatment site, and/or higher local concentrations of the antimicrobial compound at the treatment site. Moreover, the vapor-deposition process is done without the use of an aqueous solvent to coat the glass particles. When aqueous solutions of antimicrobial salts are used to coat the glass particles, at least a portion of the glass may be hydrolyzed.

Physical vapor deposition coating is a line-of-sight surface coating technique. Accordingly, when the glass particles or fibers are porous, at least some of the inner aspects of the pores are not coated, thereby leaving the bioglass available to react with aqueous liquid (e.g., tissue fluid, serum) present in the site that is treated with the bioglass composition. Thus, the coated bioglass particles and/or fibers retain the antimicrobial properties of both the glass and the coated material.

In yet another aspect, the present disclosure provides a composition. The composition can be applied to a surface (e.g., the surface of a wound, an environmental surface), where the antimicrobial properties of the composition can facilitate the reduction of viable microorganisms and/or inhibit colonization of the surface by microorganisms. The composition comprises a suspending medium as described herein, a bioactive glass comprising B₂O₃ as described herein, and an effective amount of an antimicrobial lipid component.

The antimicrobial lipid component comprises a (C7-C12)saturated fatty acid ester of a polyhydric alcohol, a (C8-C22)unsaturated fatty acid ester of a polyhydric alcohol, a (C7-C 12)saturated fatty ether of a polyhydric alcohol, a (C8-C22)unsaturated fatty ether of a polyhydric alcohol, an alkoxylated derivative thereof, or combinations thereof, wherein the alkoxylated derivative has less than 5 moles of alkoxide per mole of polyhydric alcohol; with the proviso that for polyhydric alcohols other than sucrose, the esters comprise monoesters and the ethers comprise monoethers, and for sucrose the esters comprise monoesters, diesters, or combinations thereof, and the ethers comprise monoethers, diethers, or combinations thereof. In any embodiment, the antimicrobial lipid component is present in the composition in a portion that constitutes about 0.1 percent by weight to about 20 percent by weight of the composition. In any embodiment, the antimicrobial lipid component is present in the composition in a portion that constitutes about 1 percent by weight to about 10 percent by weight of the composition. In any embodiment, the antimicrobial lipid component is present in the composition in a portion that constitutes about 2 percent by weight to about 5 percent by weight of the composition.

Antimicrobial lipid components that are suitable for use in a composition of the present disclosure are described in U.S. Patent Application Publication No. US 2005/00586 73. Non-limiting examples of suitable antimicrobial lipid components include glycerol monolaurate, glycerol monocaprate, glycerol monocaprylate, propylene glycol monolaurate, propylene glycol monocaprate, propylene glycol monocaprylate, and combinations of any two or more of the foregoing antimicrobial lipid components.

In yet another aspect, the present disclosure provides a wound dressing comprising any embodiment of the composition according to the present disclosure. The wound dressing may be any of a variety of wound dressings that are known in the art. For example, in any embodiment, the wound dressing (not shown) may comprise an absorbent layer or pad (e.g., a gauze pad or open-cell foam pad) having a patient-facing first side and a second side opposite the first side. In any embodiment, the composition may be disposed (e.g., as a coating) on the first side of the layer or pad. In any embodiment, the dressing further may comprise a water-impervious backing layer that functions to protect the absorbent layer from external moisture and/or contamination. In any embodiment, the backing layer may comprise an adhesive layer to secure the dressing to the skin proximate (e.g., surrounding) a wound site.

Compositions of the present disclosure can be used to treat biofilms in order to effect a reduction in the number of viable microorganisms in the biofilm. The biofilm may be present on inanimate surfaces (e.g., a floor, a drain, a sink, a bedrail, a tray, a medical device, a countertop, food-processing equipment, a wall) or may be present on a surface (e.g., a wound surfacc, skin, a mucous membrane) of a living organism (e.g., an animal or a plant). Thus, in yet another aspect, the present disclosure provides a method of treating a biofilmon a surface of an inanimate object. The method comprises contacting the biofilm with any embodiment of the compositions disclosed herein. Contacting the biofilm with the composition can comprise applying the composition directly onto a surface on which a biofilm is disposed. Alternatively or additionally, in any embodiment, contacting the biofilm with the composition can comprise contacting the biofilm with an article (e.g., a film, a pad, a plastic foam, a woven or nonwoven fabric, a collection of fibers, etc.) that is coated and/or imbued with any embodiment of the compositions according to the present disclosure.

Contacting the biofilm with the composition comprises contacting the biofilm with the composition for a period of time sufficient to reduce the number of viable microorganisms present in the biofilm. In any embodiment, reducing the number of microorganisms comprises reducing the number of viable microorganisms by at least about 90% (i.e., a 1-log kill) up to about 99% (i.e., a 2-log kill), up to about 99.9% (i.e., a 3-log kill), up to about 99.99% (i.e., a 4-log kill) or greater than 99.99%. In any embodiment, contacting the biofilm with the composition for a period of time sufficient to reduce the number of viable microorganisms can comprise contacting the biofilm with the composition for a period of up to about 30 minutes, about 1 hour, about 2 hours, about 3 hours, about 6 hours, about 8 hours, about 12 hours, about 15 hours, about 18 hours, about 24 hours, about 48 hours, about 72 hours, or about 96 hours. In any embodiment, contacting the biofilm with the composition for a period of time sufficient to reduce the number of viable microorganisms can comprise contacting the biofilm with the composition for a period up to about 7 days. Advantageously, contact times of up to about 7 days can be effective to treat chronic wounds or burn wounds. Assessing a reduction in the number of viable microorganisms in the biofilm can be performed using a variety of methods known in the art. As one example, assessing a reduction in the number of viable microorganisms in the biofilm can be performed as described in the Examples under the heading "Test Methods".

Compositions of the present disclosure can be used for treating a surface to inhibit (e.g., retard) or prevent formation of a biofilm thereon. Biofilm formation may be inhibited on inanimate surfaces (e.g., a floor, a drain, a sink, a bedrail, a tray, a medical device, a countertop, food-processing equipment, a wall) or may be inhibited on a surface (e.g., a wound surface, skin, a mucous membrane) of a living organism (e.g., an animal or a plant). Thus, in yet another aspect, the present disclosure provides a method of treating a surface of an inanimate object to inhibit formation of a biofilm. The method comprises contacting the surface with any embodiment of the compositions disclosed herein. Contacting the surface with the composition can comprise applying the composition (e.g., as a coating) directly onto a surface on which the operator desires to prevent formation of a biofilm. In any embodiment, contacting the surface with the composition can comprise contacting the surface with an article (e.g., a film, a pad, a plastic foam, a woven or nonwoven fabric, a collection of fibers, etc.) that is coated and/or imbued with any embodiment of the compositions according to the present disclosure.

Compositions of the present disclosure are particularly useful for treating a wound site (e.g., an incisional wound, a chronic wound, a burn wound, acute wound, trauma wound, etc.), as disclosed hereinabove. This may comprise contacting the wound with any embodiment of the compositions disclosed herein. Contacting the wound with the composition can comprise applying the composition (e.g., as a coating) directly onto a surface on which the operator desires to prevent formation of a biofilm. In any embodiment, contacting the surface with the composition can comprise contacting the surface with an article (e.g., a film, a pad, a plastic foam, a woven or nonwoven fabric, a collection of fibers, etc.) that is coated and/or imbued with any embodiment of the compositions according to the present disclosure. In any embodiment, the article may comprise a water-impervious backing layer to cover the article and the wound site. In any embodiment, after contacting the wound with the composition, the method further comprises covering the wound site with a dressing (e.g., a dressing with a water-impervious backing layer, optionally including an adhesive layer that can secure the dressing to a skin surface. In any embodiment, contacting the wound site with the composition comprises contacting the wound site with a dressing comprising the composition.

Contacting the wound with the composition comprises contacting the wound with the composition for a period of time sufficient to reduce the number of viable microorganisms present in the wound and/or to inhibit the propagation of microorganisms at the wound site. In any embodiment, contacting the wound with the composition for a period of time sufficient to reduce the number of viable microorganisms present in the wound and/or to inhibit the propagation of microorganisms at the wound site can comprise contacting the wound with the composition for a period of about 30 minutes, about 1 hour, about 2 hours, about 3 hours, about 6 hours, about 8 hours, about 12 hours, about 15 hours, about 18 hours, about 24 hours, about 48 hours, about 72 hours, or about 96 hours. In any embodiment, contacting the wound with the composition for a period of time sufficient to reduce the number of viable microorganisms can comprise contacting the wound with the composition for a period up to about 7 days. In any embodiment, optionally, the method further can comprise cleaning the wound. Cleaning the wound comprises, for example, washing, scrubbing, wiping, rinsing, debriding (e.g., surgical, mechanical, or autolytic debridement) or a combination of any two or more of the foregoing processes.

### EXEMPLARY EMBODIMENTS

Embodiment A is a composition, comprising:
   a suspending medium;
   a bioactive glass comprising B₂O₃; and
   an effective amount of an antimicrobial biguanide compound.
Embodiment B is the composition of Embodiment A, wherein the antimicrobial biguanide is selected from the group consisting of polyhexamethylene biguanide, chlorhexidine, alexidine, polyaminopropyl biguanide, a salt of any one of the foregoing antimicrobial biguanides, and a combination of any two or more of the foregoing antimicrobial biguanides.
Embodiment C is the composition of Embodiment A or Embodiment B, wherein the antimicrobial biguanide is present in the composition in a portion that constitutes about 0.01 percent by weight to about 5 percent by weight of the composition.
Embodiment D is a composition, comprising:
   a suspending medium;
   a bioactive glass comprising B₂O₃; and
   an effective amount of an antimicrobial quaternary ammonium compound.
Embodiment E is the composition of Embodiment D, wherein the antimicrobial quaternary ammonium compound is selected from the group consisting of cetylpyridinium chloride, benzalkonium chloride, benzethonium chloride, methylbenzethonium chloride, cetalkonium chloride, cetylpyridinium chloride, cetrimonium, cetrimide, dofanium chloride, tetraethylammonium bromide, didecyldimethylammonium chloride, domiphen bromide, and a combination of any two or more of the foregoing antimicrobial quaternary ammonium compounds.
Embodiment F is the composition of Embodiment D or Embodiment E, wherein the antimicrobial quaternary ammonium compound is present in the composition in a portion that constitutes about 0.01 percent by weight to about 5 percent by weight of the composition.
Embodiment G is a reference composition, comprising:
   a suspending medium;
   a bioactive glass comprising B₂O₃, the bioactive glass having a surface; and an effective amount of an antimicrobial compound that comprises silver or copper;
   wherein the antimicrobial compound is present in the composition as a coating on the surface of the bioactive glass.
Embodiment H is the reference composition of Embodiment G, wherein the antimicrobial compound is selected from the group consisting of silver oxide, metallic silver, and combinations thereof.
Embodiment I is the reference composition of Embodiment G or Embodiment H, wherein the surface layer is deposited using a process selected from the group consisting of solution deposition and vapor deposition.
Embodiment J is the composition of Embodiment 1, wherein the vapor deposition process comprises magnetron sputtering, thermal evaporation, e-beam evaporation, ion beam sputtering, and cathodic arc deposition.
Embodiment K is a composition, comprising:
   a suspending medium;
   a bioactive glass comprising B₂O₃; and
   an effective amount of an antimicrobial lipid component;
   wherein the antimicrobial lipid component comprises a (C7-C12)saturated fatty acid ester of a polyhydric alcohol, a (C8-C22)unsaturated fatty acid ester of a polyhydric alcohol, a (C7-C12)saturated fatty ether of a polyhydric alcohol, a (C8-C22)unsaturated fatty ether of a polyhydric alcohol, an alkoxylated derivative thereof, or combinations thereof, wherein the alkoxylated derivative has less than 5 moles of alkoxide per mole of polyhydric alcohol; with the proviso that for polyhydric alcohols other than sucrose, the esters comprise monoesters and the ethers comprise monoethers, and for sucrose the esters comprise monoesters, diesters, or combinations thereof, and the ethers comprise monoethers, diethers, or combinations thereof.
Embodiment L is the composition of Embodiment K, wherein the antimicrobial lipid component includes a compound selected from the group consisting of glycerol monolaurate, glycerol monocaprate, glycerol monocaprylate, propylene glycol monolaurate, propylene glycol monocaprate, propylene glycol monocaprylate, or combinations thereof.
Embodiment M is the composition of Embodiment K or Embodiment L, wherein the antimicrobial lipid component is present in the composition in a portion that constitutes about 0.1 percent by weight to about 20 percent by weight of the composition.
Embodiment N is the composition of any one of the preceding Embodiments, wherein the suspending medium comprises a first polyol with a melting point less than or equal to 25° C.
Embodiment O is the composition of Embodiment N, wherein the suspending medium further comprises a second polyol with a melting point higher than 25° C.
Embodiment P is the composition of any one of the preceding Embodiments, wherein the suspending medium includes a polyol selected from the group consisting of PEG 400, glycerol, and combinations thereof.
Embodiment Q is the composition of any one of the preceding Embodiments, wherein the composition has a viscosity of about 0.1 to about 100,000 Pa·s at 37 degrees C measured at a shear rate of 3.8/s in parallel plate mode.
Embodiment R is the composition of any one of the preceding Embodiments, wherein the bioactive glass comprises less than 35 weight percent SiO₂.
Embodiment S is the composition of any one of the preceding claims, wherein the bioactive glass comprises about 1 weight percent to about 70 weight percent B₂O₃.
Embodiment T is the composition of Embodiment S, wherein the bioactive glass comprises about 15 weight percent to about 60 weight percent B₂O₃.
Embodiment U is the composition of any one of the preceding Embodiments, wherein the bioactive glass comprises bioactive fiberglass, bioactive glass particles, or combinations thereof.
Embodiment V is the composition of Embodiment U, wherein the bioactive glass particles comprise non-interlinked bioactive glass particles.
Embodiment W is the composition of Embodiment U or Embodiment V, wherein the glass particles, if present, have a median diameter of about 0.5 microns to about 100 microns.
Embodiment X is the composition of any one of the preceding Embodiments, wherein the bioactive glass is present in the composition in a portion that constitutes about 5 percent by weight to about 95 percent by weight.
Embodiment Y is the composition of any one of the preceding Embodiments, wherein the suspending medium is a pharmaceutically acceptable suspending medium.
Embodiment Z is the composition of Embodiment Y, wherein the pharmaceutically acceptable suspending medium does not cause undue toxicity to soft tissue in contact with the suspending medium.
Embodiment AA is a wound dressing comprising the composition of any one of the preceding claims.
Embodiment AB is a method of treating a biofilm, on a surface of an inanimate object, the method comprising contacting
   the biofilm with the composition of any one of Embodiments A through Z.
Embodiment AC is the method of Embodiment AB, wherein contacting the biofilm with the composition comprises contacting the biofilm with the composition for a period of time up to seven days.
Embodiment AD is the composition of any one of Embodiments A through Z for use in the treatment of a wound site, wherein the use comprises contacting the wound site with the composition.
Embodiment AE is the composition of Embodiment AD, further comprising, after contacting the wound site with the composition, covering the wound site with a dressing.
Embodiment AF is the composition for use of Embodiment AD, wherein contacting the wound site with the composition comprises contacting the wound site with a dressing comprising the composition.
Embodiment AG is the composition for use of any one of Embodiments AD through AF, wherein contacting the wound site with the composition comprises contacting the wound site with the composition for a period of time up to seven days.

### EXAMPLES

Objects and advantages of this invention are further illustrated by the following examples, but the particular materials and amounts thereof recited in these examples, as well as other conditions and details, should not be construed to unduly limit this invention. Unless otherwise indicated, all parts and percentages are on a weight basis, all water is distilled water, and all molecular weights are weight average molecular weight.

Raw materials utilized in the sample preparation are shown in Table 1.

**Table 1. Materials**

| **Component** | **Description** | **Supplier** |
|---|---|---|
| TSB | Bacto™ tryptic soy broth | Becton, Dickinson, and Company, Sparks, MD |
| Agar | Bacto™ agar | Becton, Dickinson, and Company, Sparks, MD |
| NaCl | Sodium chloride | BDH Chemicals, West Chester, PA |
| KCl | Potassium chloride | BDH Chemicals, West Chester, PA |
| Na₂HPO₄ | Sodium hydrogen phosphate | Avantor Performance Materials, Center Valley, PA |
| KH₂PO₄ | Potassium hydrogen phosphate | EDM Chemicals, Gibbstown, NJ |
| D/E neutralizing broth | Difco D/E (Dey/Engley) neutralizing broth | Becton, Dickinson, and Company, Sparks, MD |
| Butterfield's buffer | Mini Flip-Top Vial with Butterfield's Buffer | 3M Company, St. Paul, MN |
| PEG400 | Poly (ethylene glycol) 400 NF | Spectrum, New Brunswick, NJ |
| PEG4000 | Poly (ethylene glycol) 4000 | Merck KGaA, 64271 Darmstadt, Germany |
| PHMB | Cosmocil® CQ (20% polyhexamethylene biguanide) | Arch Chemicals, Norwalk, CT |
| OCT | Octenidine dihydrochloride | Dishman Pharmaceuticals and Chemicals, Ahmedabad, India. |
| BKC | Benzalkonium chloride | Spectrum, New Brunswick, NJ |
| CPC | Cetylpyridinium chloride | Sigma-Aldrich, St. Louis, MO |
| CHG | Chlorhexidine gluconate | Medichem; Barcelona, Spain |
| PG8 | Capmul® PG-8 propylene glycol monocaprylate | Abitec, Columbus, OH |

### Preparation of Bioglass Particles

Borate bioglass frits having the 13-93B3 glass composition were prepared and ground into glass particlcs essentially as described by Dcliormanh and M.N. Rahaman (Advances in Bioceramics and Porous Ceramics V; Vol. 33, page 12) except that grinding conditions and classification procedures were adjusted to that the median particle diameter was about 16 microns as measured by laser adsorption.

### Test Methods

### Inhibition of Microbial Growth

### Mature Colony

A culture of *Pseudomonas aeruginosa* ATCC #15442 was grown for 20 ± 2 hours in TSB at 37°C with agitation at 225 rpm. The culture was diluted 1:10,000 in phosphate-buffered saline ("PBS"; 137mM NaCl, 2.7 mM KCl, 10 mM Na₂HPO₄, 1.8 mM KH₂PO₄) and 10 µl of the diluted culture was spotted onto 0.2 µm polycarbonate membrane filters (25 mm Whatman™ Nucleopore™ Track-Etch membranes) placed on TSB agar plates (1.5% agar in TSB, autoclaved, cooled to 55°C, poured into petri dish, and cooled to room temperatures). The plates were incubated at 37°C for 24 hours.

After incubation, the membranes containing a biofilm were transferred to 6-well plates containing TSB agar. One hundred milligrams of Example Formulations were transferred onto the biofilm and incubated at 37°C for 18 hours. Each Example Formulation was tested in triplicate along with a control sample containing no antimicrobial. After exposure, the membrane containing the Example Formulation and biofilm was transferred to a 50 ml conical tube containing 10 ml D/E neutralization broth. The samples were briefly mixed and placed in a floating rack in a sonicating water bath. All recovered samples were sonicated for 1 minute in room temperature water followed by 1 minute of vortexing. The bacterial suspension was diluted 1:10 in Butterfield's buffer, and 1 ml of each dilution was plated onto a Petrifilm™ Aerobic Count plate ("AC" plate, obtained from 3M Company, St. Paul, MN). The plates were incubated for 24 to 48 hours at 37°C. The colony forming units (CFUs) were enumerated according to the manufacturer's instructions.

The log reduction value (LRV) for each formulation was calculated as the difference between the mean log CFU of the biofilms exposed to the control material containing no antimicrobial (control formulation) and the mean log CFU of the biofilms exposed to the antimicrobial composition (Example formulation).

### Immature Colony

This model was similar to the mature colony biofilm model (above) with the following modifications:
1) The membranes inoculated with 10 µl of the 1:10,000 diluted culture were incubated for 6 hours instead of 24 hours.
2) The biofilms exposed to antimicrobial materials were incubated for 48 hours instead of 18 hours.

The log reduction value (LRV) for each formulation was calculated as the difference between the mean log CFU of the biofilms exposed to the control material containing no antimicrobial (control formulation) and the mean log CFU of the biofilms exposed to the antimicrobial composition (Example formulation).

### Examples 1-16

Examples 1 through 12 ("E1" through "E12", respectively) were prepared by weighing the components (listed in Table 3) in a Max 20 cup and mixing 2 times for 30 seconds at 2,500 rpm in a DAC 150.1 FVZ SpeedMixer™ (FlackTek Inc., Landrum, SC).

Examples 13 through 16 ("E13" through "E16", respectively) utilized bioglass that was coated with silver. The apparatus and method for coating the bioglass is described in U.S.

Patent No. 7,727,931 (Brey et. al.) and is shown in Figures 1 and 2 of that patent. For E-13, 32.0g of the borate bioglass particles were placed into the particle agitator apparatus and the chamber was then evacuated. Once the chamber pressure was approximately 10⁻⁵ torr, argon and oxygen sputtering gas was admitted into the chamber. Argon flow rate was 80 sccm (standard cubic centimeter per minute), and oxygen flow rate was 10 sccm. The chamber pressure was adjusted to approximately 10 millitorr. The silver deposition process was then started by applying cathodic power. The particle agitator shaft was rotated at approximately 6 rpm during the silver deposition process. The chamber was backfilled with air and the silver coated particles were removed from the apparatus. The silver sputter target was weighed before and after the coating. The weight loss of silver was used to determine the amount of silver deposited using capture efficiency of 6%. The silver coated bioglasses are described in Table 2.

After coating the bioglass with silver, E-13 through E-16 were mixed and prepared as described for E-1. Example formulation compositions are shown in Table 3.

**Table 2: Silver Coated Bioglass (reference)**

| **Silver Bioglass (SB)** | **Cathodic Power (kW)** | **Sputtering Time (hours)** | **Silver Weight Loss (g)** | **Silver on Bioglass (wt. %)** |
|---|---|---|---|---|
| SB-1 | 0.03 | 3 | 2.11 | 0.4 |
| SB-2 | 0.10 | 3 | 6.11 | 1.1 |
| SB-3 | 0.20 | 3 | 11.9 | 2.2 |
| SB-4 | 0.20 | 6 | 23.3 | 4.4 |

**Table 3 Example Formulations**

| **Example** | **Borate Bioglass Particles (%)** | **PEG 4000 (%)** | **PEG 400 (%)** | **Antimicrobial** | | **Water (%)** |
|---|---|---|---|---|---|---|
| | | | | | **(%)** | |
| E-1 | 70.0 | 4.5 | 20.5 | PG8 | 5.0 | 0 |
| E-2 | 10.0 | 16.2 | 73.6 | PHMB | 0.1 | 0.2 |
| E-3 | 20.0 | 14.4 | 65.4 | PHMB | 0.1 | 0.2 |
| E-4 | 40.0 | 10.8 | 49.0 | PHMB | 0.1 | 0.2 |
| E-5 | 70.0 | 5.4 | 24.4 | PHMB | 0.1 | 0.2 |
| E-6 | 40.0 | 10.7 | 48.8 | PHMB | 0.1 | 0.4 |
| E-7 | 40.0 | 10.7 | 48.8 | CHG | 0.1 | 0.4 |
| E-8 | 40.0 | 10.1 | 45.9 | OCT | 0.1 | 3.9 |
| E-9 | 40.0 | 9.9 | 45.1 | BKC | 0.1 | 4.9 |
| E-10 | 40.0 | 10.7 | 48.8 | CPC | 0.1 | 0.4 |
| E-11 | 40.0 | 9.9 | 45.1 | PG8 | 5.0 | 0 |
| E-12 | 40.0 | 10.7 | 48.8 | PHMB | 0.1 | 0.4 |
| E-13 | 39.6[a] | 10.7 | 48.8 | PHMB | 0.1 | 0.4 |
| E-14 | 38.8[b] | 10.7 | 48.8 | PHMB | 0.1 | 0.4 |
| E-15 | 37.8[c] | 10.7 | 48.8 | PHMB | 0.1 | 0.4 |
| E-16 | 35.6[d] | 10.7 | 48.8 | PHMB | 0.1 | 0.4 |

| | | | | | | |
|---|---|---|---|---|---|---|
| [a] SB-1 [b] SB-2 [c] SB-3 [d] SB-4 | | | | | | |

### Results

Inhibition of microbial growth was measured for each of the Example formulations. Results are shown in Table 4, with the associated control compositions.

**Table 4: Test Results**

| **Formulation** | **Control** | **LRV** |
|---|---|---|
| Immature Biofilm | | |
| E-1 | [a] | 3.77 [c] |
| E-2 | [a] | 3.02 [c] |
| E-3 | [a] | 3.74 [c] |
| E-4 | [a] | 3.74 [c] |
| E-5 | [a] | 3.74 [c] |

| Mature Biofilm | | |
|---|---|---|
| E-6 | [b] | 5.00 [c] |
| E-7 | [b] | 4.74 [c] |
| E-8 | [b] | 4.74 [c] |
| E-9 | [b] | 4.74 [c] |
| E-10 | [b] | 2.09 |
| E-11 | [b] | 3.91 |
| E-12 | [b] | 3.24 |
| E-13 | [b] | 6.05 |
| E-14 | [b] | 5.76 |
| E-15 | [b] | 7.23 [c] |
| E-16 | [b] | 5.90 |

| | | |
|---|---|---|
| [a] 74% PEG4000 / 26% water (LRV was -5.13) [b] 17.93 PEG4000 / 81.67 PEG 400 / 0.4% water (LRV was 0) [c] below level of detection | | |

## Claims

1. A composition, comprising:
a suspending medium;
a bioactive glass comprising B₂O₃; and
an effective amount of an antimicrobial biguanide compound.

2. The composition of claim 1, wherein the antimicrobial biguanide is selected from the group consisting of polyhexamethylene biguanide, chlorhexidine, alexidine, polyaminopropyl biguanide, a salt of any one of the foregoing antimicrobial biguanides, and a combination of any two or more of the foregoing antimicrobial biguanides.

3. The composition of claim 1 or claim 2, wherein the antimicrobial biguanide is present in the composition in a portion that constitutes about 0.01 percent by weight to about 5 percent by weight of the composition.

4. A composition, comprising:
a suspending medium;
a bioactive glass comprising B₂O₃; and
an effective amount of an antimicrobial quaternary ammonium compound.

5. A composition, comprising:
a suspending medium;
a bioactive glass comprising B₂O₃; and
an effective amount of an antimicrobial lipid component;
wherein the antimicrobial lipid component comprises a (C7-C12)saturated fatty acid ester of a polyhydric alcohol, a (C8-C22)unsaturated fatty acid ester of a polyhydric alcohol, a (C7-C12)saturated fatty ether of a polyhydric alcohol, a (C8-C22)unsaturated fatty ether of a polyhydric alcohol, an alkoxylated derivative thereof, or combinations thereof, wherein the alkoxylated derivative has less than 5 moles of alkoxide per mole of polyhydric alcohol; with the proviso that for polyhydric alcohols other than sucrose,
the esters comprise monoesters and the ethers comprise monoethers, and for sucrose the esters comprise monoesters, diesters, or combinations thereof, and the ethers comprise monoethers, diethers, or combinations thereof.

6. The composition of claim 5, wherein the antimicrobial lipid component includes a compound selected from the group consisting of glycerol monolaurate, glycerol monocaprate, glycerol monocaprylate, propylene glycol monolaurate, propylene glycol monocaprate, propylene glycol monocaprylate, or combinations thereof.

7. The composition of claim 5 or claim 6, wherein the antimicrobial lipid component is present in the composition in a portion that constitutes about 0.1 percent by weight to about 20 percent by weight of the composition.

8. The composition of any one of the preceding claims, wherein the suspending medium comprises a first polyol with a melting point less than or equal to 25° C.

9. The composition of any one of the preceding claims, wherein the composition has a viscosity of about 0.1 to about 100,000 Pa·s at 37 degrees C measured at a shear rate of 3.8/s in parallel plate mode.

10. The composition of any one of the preceding claims, wherein the bioactive glass comprises less than 35 weight percent SiO₂.

11. The composition of any one of the preceding claims, wherein the bioactive glass comprises about 1 weight percent to about 70 weight percent B₂O₃.

12. The composition of any one of the preceding claims, wherein the bioactive glass comprises bioactive fiberglass, bioactive glass particles, or combinations thereof.

13. The composition of any one of the preceding claims, wherein the bioactive glass is present in the composition in a portion that constitutes about 5 percent by weight to about 95 percent by weight.

14. A wound dressing comprising the composition of any one of the preceding claims.

15. A method of treating a biofilm on a surface of an inanimate object, the method comprising contacting the biofilm with the composition of any one of claims 1 through 13.

16. A method of treating a surface of an inanimate object to inhibit formation of a biofilm thereon, the method comprising contacting the surface with the composition of any one of claims 1 through 13.

17. The composition of any one of claims 1 through 13 for use in the treatment of a wound site.

18. The composition for use of claim 17, wherein the composition is comprised in a dressing.

## Patentansprüche

1. Eine Zusammensetzung, umfassend:
ein Suspensionsmedium;
ein bioaktives Glas, umfassend B₂O₃; und
eine wirksame Menge einer antimikrobiellen Biguanidverbindung.

2. Die Zusammensetzung nach Anspruch 1, wobei das antimikrobielle Biguanid ausgewählt ist aus der Gruppe bestehend aus Polyhexamethylenbiguanid, Chlorhexidin, Alexidin, Polyaminopropylbiguanid, einem Salz von einem beliebigen der vorhergehenden antimikrobiellen Biguanide und einer Kombination von zwei oder mehreren der vorhergehenden antimikrobiellen Biguanide.

3. Die Zusammensetzung nach Anspruch 1 oder 2, wobei das antimikrobielle Biguanid in der Zusammensetzung in einer Menge vorliegt, die etwa 0,01 Gewichtsprozent bis etwa 5 Gewichtsprozent der Zusammensetzung ausmacht.

4. Eine Zusammensetzung, umfassend:
ein Suspensionsmedium;
ein bioaktives Glas, umfassend B₂O₃; und
eine wirksame Menge einer antimikrobiellen quaternären Ammoniumverbindung.

5. Eine Zusammensetzung, umfassend:
ein Suspensionsmedium;
ein bioaktives Glas, umfassend B₂O₃; und
eine wirksame Menge eines antimikrobiellen Lipidbestandteils;
wobei der antimikrobielle Lipidbestandteil einen (C7-C12)-gesättigten Fettsäureester eines mehrwertigen Alkohols, einen (C8-C22)-ungesättigten Fettsäureester eines mehrwertigen Alkohols, einen (C7-C12)-gesättigten Fettether eines mehrwertigen Alkohols, einen (C8-C22)-ungesättigten Fettether eines mehrwertigen Alkohols, ein alkoxyliertes Derivat davon oder
Kombinationen davon umfasst, wobei das alkoxylierte Derivat weniger als 5 Mol Alkoxid pro Mol mehrwertigen Alkohols aufweist; mit der Maßgabe, dass für mehrwertige Alkohole außer Saccharose die Ester Monoester umfassen und die Ether Monoether umfassen, und für Saccharose die Ester Monoester, Diester oder Kombinationen davon umfassen, und die Ether Monoether, Diether oder Kombinationen davon umfassen.

6. Die Zusammensetzung nach Anspruch 5, wobei der antimikrobielle Lipidbestandteil eine Verbindung beinhaltet, die ausgewählt ist aus der Gruppe bestehend aus Glycerinmonolaurat, Glycerinmonocaprat, Glycerinmonocaprylat, Propylenglykolmonolaurat, Propylenglykolmonocaprat, Propylenglykolmonocaprylat oder Kombinationen davon.

7. Die Zusammensetzung nach Anspruch 5 oder 6, wobei der antimikrobielle Lipidbestandteil in der Zusammensetzung in einer Menge vorliegt, die etwa 0,1 Gewichtsprozent bis etwa 20 Gewichtsprozent der Zusammensetzung ausmacht.

8. Die Zusammensetzung nach einem der vorstehenden Ansprüche, wobei das Suspensionsmedium ein erstes Polyol mit einem Schmelzpunkt von weniger als oder gleich 25 °C umfasst.

9. Die Zusammensetzung nach einem der vorstehenden Ansprüche, wobei die Zusammensetzung eine Viskosität von etwa 0,1 bis etwa 100.000 Pa·s bei 37 °C aufweist, gemessen bei einer Scherrate von 3,8/s im Parallelplattenmodus.

10. Die Zusammensetzung nach einem der vorstehenden Ansprüche, wobei das bioaktive Glas weniger als 35 Gewichtsprozent SiO₂ umfasst.

11. Die Zusammensetzung nach einem der vorstehenden Ansprüche, wobei das bioaktive Glas etwa 1 Gewichtsprozent bis etwa 70 Gewichtsprozent B₂O₃ umfasst.

12. Die Zusammensetzung nach einem der vorstehenden Ansprüche, wobei das bioaktive Glas bioaktives Glasfaserglas, bioaktive Glaspartikel oder Kombinationen davon umfasst.

13. Die Zusammensetzung nach einem der vorstehenden Ansprüche, wobei das bioaktive Glas in der Zusammensetzung in einer Menge vorliegt, die etwa 5 Gewichtsprozent bis etwa 95 Gewichtsprozent ausmacht.

14. Ein Wundverband, umfassend die Zusammensetzung nach einem der vorstehenden Ansprüche.

15. Ein Verfahren zur Behandlung eines Biofilms auf einer Oberfläche eines nicht lebenden Objekts, das Verfahren umfassend das Inkontaktbringen des Biofilms mit der Zusammensetzung nach einem der Ansprüche 1 bis 13.

16. Ein Verfahren zur Behandlung einer Oberfläche eines nicht lebenden Objekts, um die Bildung eines Biofilms darauf zu hemmen, das Verfahren umfassend das Inkontaktbringen der Oberfläche mit der Zusammensetzung nach einem der Ansprüche 1 bis 13.

17. Die Zusammensetzung nach einem der Ansprüche 1 bis 13 zur Verwendung bei der Behandlung einer Wundstelle.

18. Die Zusammensetzung zur Verwendung nach Anspruch 17, wobei die Zusammensetzung in einem Verband enthalten ist.

## Revendications

1. Composition comprenant :
un milieu de suspension ;
un verre bioactif comprenant B₂O₃ ; et
une quantité efficace d'un composé biguanide antimicrobien.

2. Composition selon la revendication 1, dans laquelle le biguanide antimicrobien est choisi dans le groupe constitué de polyhexaméthylène biguanide, chlorhexidine, alexidine, polyaminopropyl-biguanide, un sel de l'un quelconque des biguanides antimicrobiens qui précèdent et une combinaison de n'importe quels biguanides antimicrobiens qui précèdent au nombre de deux ou plus.

3. Composition selon la revendication 1 ou la revendication 2, dans laquelle le biguanide antimicrobien est présent dans la composition dans une partie qui constitue environ 0,01 pour cent en poids à environ 5 pour cent en poids de la composition.

4. Composition comprenant :
un milieu de suspension ;
un verre bioactif comprenant B₂O₃ ; et
une quantité efficace d'un composé d'ammonium quaternaire antimicrobien.

5. Composition comprenant :
un milieu de suspension ;
un verre bioactif comprenant B₂O₃ ; et
une quantité efficace d'un composant lipidique antimicrobien ;
dans laquelle le composant lipidique antimicrobien comprend un ester d'acide gras saturé en (C7 à C12) d'un alcool polyhydrique, un ester d'acide gras insaturé en (C8 à C22) d'un alcool polyhydrique, un éther gras saturé en (C7 à C12) d'un alcool polyhydrique, un éther gras insaturé en (C8 à C22) d'un alcool polyhydrique, un dérivé alcoxylé de ceux-ci, ou des combinaisons de ceux-ci, dans laquelle le dérivé alcoxylé a moins de 5 moles d'alcoolate par mole d'alcool polyhydrique ; à condition que pour des alcools polyhydriques autres que le saccharose, les esters comprennent des monoesters et les éthers comprennent des monoéthers, et pour le saccharose les esters comprennent des monoesters, diesters, ou des combinaisons de ceux-ci, et les éthers comprennent des monoéthers, diéthers, ou des combinaisons de ceux-ci.

6. Composition selon la revendication 5, dans laquelle le composant lipidique antimicrobien inclut un composé choisi dans le groupe constitué de monolaurate de glycérol, monocaprate de glycérol, monocaprylate de glycérol, monolaurate de propylène glycol, monocaprate de propylène glycol, monocaprylate de propylène glycol, ou des combinaisons de ceux-ci.

7. Composition selon la revendication 5 ou la revendication 6, dans laquelle le composant lipidique antimicrobien est présent dans la composition dans une partie qui constitue environ 0,1 pour cent en poids à environ 20 pour cent en poids de la composition.

8. Composition selon l'une quelconque des revendications précédentes, dans laquelle le milieu de suspension comprend un premier polyol avec un point de fusion inférieur ou égal à 25 °C.

9. Composition selon l'une quelconque des revendications précédentes, dans laquelle la composition a une viscosité d'environ 0,1 à environ 100 000 Pa·s à 37 degrés C mesurée à un taux de cisaillement de 3,8/s en mode plaques parallèles.

10. Composition selon l'une quelconque des revendications précédentes, dans laquelle le verre bioactif comprend moins de 35 pour cent en poids de SiO₂.

11. Composition selon l'une quelconque des revendications précédentes, dans laquelle le verre bioactif comprend environ 1 pour cent en poids à environ 70 pour cent en poids de B₂O₃.

12. Composition selon l'une quelconque des revendications précédentes, dans laquelle le verre bioactif comprend de la fibre de verre bioactive, des particules de verre bioactives, ou des combinaisons de celles-ci.

13. Composition selon l'une quelconque des revendications précédentes, dans laquelle le verre bioactif est présent dans la composition dans une partie qui constitue environ 5 pour cent en poids à environ 95 pour cent en poids.

14. Pansement pour plaie comprenant la composition selon l'une quelconque des revendications précédentes.

15. Procédé de traitement d'un film biologique sur une surface d'un objet inanimé, le procédé comprenant la mise en contact du film biologique avec la composition selon l'une quelconque des revendications 1 à 13.

16. Procédé de traitement d'une surface d'un objet inanimé pour inhiber la formation d'un film biologique sur celle-ci, le procédé comprenant la mise en contact de la surface avec la composition selon l'une quelconque des revendications 1 à 13.

17. Composition selon l'une quelconque des revendications 1 à 13 pour utilisation dans le traitement d'un site de plaie.

18. Composition pour utilisation selon la revendication 17, dans laquelle la composition est comprise dans un pansement.
